# EUROPEAN PATENT APPLICATION

(11) **EP 4 492 386 A1**
(43) Date of publication of application: **15.01.2025**
(21) Application number: 23766659.9
(22) Date of filing: 01.03.2023
(51) Int. Cl.: G16B 25/20

(54) **METHOD, DEVICE, AND PROGRAM**

(30) Priority: 09.03.2022 JP 2022036263
(71) Applicant: FUJIFILM Corporation, Tokyo 106-8620 (JP)
(72) Inventor: NAGASE, Masaya, Ashigarakami-gun, Kanagawa 258-8538 (JP)
(74) Representative: Dehns Germany Partnerschaft mbB
(86) International application number: PCT/JP2023/007518
(87) International publication number: WO 2023/171482

(57) **Abstract**

Provided are a method, an apparatus, and a program capable of measuring as many feature amounts as possible. In a method of selecting one or a plurality of feature amounts that are used to predict or discriminate a characteristic of a sample and designing one or a plurality of measurers that measure the feature amount, the method performed by a processor includes a step of attempting to design the measurer for a feature amount candidate, an evaluation step of evaluating interaction between the designed plurality of measurers, a data structure construction step of associating, as a combination pair of the measurers that are unable to be set at the same time, a combination of the measurers whose interaction evaluated in the evaluation step is problematic to construct a graph-like data structure, an extraction step of selecting a portion corresponding to one or a plurality of independent sets from the graph-like data structure to extract one or more feature amount set candidates that do not include the combination pair whose interaction is problematic, and a selection step of selecting, as a feature amount set to be measured, the feature amount set candidate or a subset of the feature amount set candidate.

## Description

### BACKGROUND OF THE INVENTION

### 1. Field of the Invention

The present invention relates to a method, an apparatus, and a program that measure, in a case where a feature amount for predicting or discriminating a characteristic of a sample is selected and a measurer that measures the feature amount is designed, as many feature amounts as possible while avoiding interaction between measurers.

### 2. Description of the Related Art

A problem of predicting or discriminating a characteristic of a sample from a feature amount is a central issue handled by modern machine learning and the like. For example, even in a case of being limited to gene analysis, there is a multi-class cancer classification problem based on a deoxyribonucleic acid (DNA) methylation pattern.

However, in practice, there are a large number of feature amounts that can be candidates, or there is a problem of measurement cost or the like. Thus, there may be a case where only a part of the feature amounts can be measured. However, in practice, not only the feature amount may be simply selected, but also design of a measurer for measuring the characteristic of the selected feature amount may be separately required.

For example, in the gene analysis, a large number of genes (feature amounts) of tens of thousands to hundreds of thousands or more can be comprehensively measured with a microarray, a sequencer, or the like. However, the number of genes required for the sample characteristic is smaller than the above large number of genes. Thus, the measurement is narrowed down by amplicon sequencing or the like. In particular, in such a case, there are examples such as designing a polymerase chain reaction (PCR) primer (measurer).

However, in a case where the PCR primer is designed, the primers may form a dimer. In this case, since interaction occurs between the measurers, there is a problem that the selected feature amounts cannot be measured at the same time.

However, in the related art, a procedure of selecting a feature amount and then designing a measurer for the selected feature amount is generally used. For example, Emese Meglecz et al., "A user-friendly program to select microsatellite markers and design primers from large sequencing projects", Bioinformatics, Volume 26, issue 3, 1 February 2010, Pages 403-404 discloses a method of collectively performing selection of a marker from sequence data and primer design. In Emese Meglecz et al., "A user-friendly program to select microsatellite markers and design primers from large sequencing projects", Bioinformatics, Volume 26, issue 3, 1 February 2010, Pages 403-404, an appropriate marker is selected from the sequence data, and a primer is designed for the marker to be presented to a user.

Further, JP2019-528729A discloses a method of deriving a prediction read from a primer for a feature amount to decide a feature amount set.

### SUMMARY OF THE INVENTION

However, in both of Emese Meglecz et al., "A user-friendly program to select microsatellite markers and design primers from large sequencing projects", Bioinformatics, Volume 26, issue 3, 1 February 2010, Pages 403-404 and JP2019-528729A, necessity of taking into consideration the interaction between the measurers, such as the dimer formation between the primers, is not mentioned or a sufficient solution is not presented. Thus, there may be a case where only a part of the feature amounts intended to be narrowed down can be measured.

The present invention has been made in view of the above-described problems, and an object of the present invention is to provide a method, an apparatus, and a program capable of measuring, in a case where a measurer needs to be designed, as many feature amounts as possible in consideration of interaction between measurers.

A method of a first aspect is a method of selecting one or a plurality of feature amounts that are used to predict or discriminate a characteristic of a sample and designing one or a plurality of measurers that measure the feature amount, in which the method performed by a processor includes a step of attempting to design the measurer for a feature amount candidate, an evaluation step of evaluating interaction between the designed plurality of measurers, a data structure construction step of associating, as a combination pair of the measurers that are unable to be set at the same time, a combination of the measurers whose interaction evaluated in the evaluation step is problematic to construct a graph-like data structure, an extraction step of selecting a portion corresponding to one or a plurality of independent sets from the graph-like data structure to extract one or more feature amount set candidates that do not include the combination pair whose interaction is problematic, and a selection step of selecting, as a feature amount set to be measured, the feature amount set candidate or a subset of the feature amount set candidate.

In the method of a second aspect, in the extraction step, one or a plurality of maximal independent sets are selected among the plurality of independent sets to extract one or more feature amount set candidates. Next to the selection step, the method further includes an operation step of performing an operation including feature amount selection for each of the selected feature amount set candidates, and a confirmation step of evaluating a result of the operation step to confirm a feature amount set to be measured based on a result of the evaluation.

In the method of a third aspect, in the step of attempting to design the measurer, the design of the plurality of measurers is attempted for at least one or more of the feature amount candidates, respectively. In the data structure construction step, all the plurality of measurers are considered and any measurer pair for the same feature amount candidate is associated as the combination pair that is unable to be set at the same time.

In the method of a fourth aspect, the graph-like data structure includes a graph data structure or a data structure conforming to a graph.

In the method of a fifth aspect, the feature amount is a gene, and the measurer is a primer.

In the method of a sixth aspect, the interaction between the measurers is a primer dimer.

In the method of a seventh aspect, the gene is a methylation DNA site or a miRNA.

An apparatus of an eighth aspect is an apparatus comprising a processor that selects one or a plurality of feature amounts that are used to predict or discriminate a characteristic of a sample and designs one or a plurality of measurers that measure the feature amount, in which the processor is configured to attempt to design the measurer for a feature amount candidate, evaluate interaction between the designed plurality of measurers, associate, as a combination pair of the measurers that are unable to be set at the same time, a combination of the measurers whose evaluated interaction is problematic to construct a graph-like data structure, select a portion corresponding to one or a plurality of independent sets from the graph-like data structure to extract one or more feature amount set candidates that do not include the combination pair whose interaction is problematic, and select, as a feature amount set to be measured, the feature amount set candidate or a subset of the feature amount set candidate.

In the apparatus of a ninth aspect, the processor is configured to select one or a plurality of maximal independent sets among the plurality of independent sets to extract one or more feature amount set candidates, perform an operation including feature amount selection for each of the extracted feature amount set candidates, and evaluate a result of an operation step to confirm a feature amount set to be measured based on a result of the evaluation.

In the apparatus of a tenth aspect, in a case of attempting to design the measurer, the design of the plurality of measurers is attempted for at least one or more of the feature amount candidates, respectively. All the plurality of measurers are considered and any measurer pair for the same feature amount candidate is associated as the combination pair that is unable to be set at the same time to construct the graph-like data structure.

A program of an eleventh aspect is a program that selects one or a plurality of feature amounts that are used to predict or discriminate a characteristic of a sample and designs one or a plurality of measurers that measure the feature amount, in which the program causes a computer to execute a step of attempting to design the measurer for a feature amount candidate, an evaluation step of evaluating interaction between the designed plurality of measurers, a data structure construction step of associating, as a combination pair of the measurers that are unable to be set at the same time, a combination of the measurers whose interaction evaluated in the evaluation step is problematic to construct a graph-like data structure, an extraction step of selecting a portion corresponding to one or a plurality of independent sets from the graph-like data structure to extract one or more feature amount set candidates that do not include the combination pair whose interaction is problematic, and a selection step of selecting, as a feature amount set to be measured, the feature amount set candidate or a subset of the feature amount set candidate.

According to the method, the apparatus, and the program of the present invention, it is possible to measure, in a case where the measurer needs to be designed, as many feature amounts as possible in one measurement in consideration of the interaction between the measurers.

### BRIEF DESCRIPTION OF THE DRAWINGS

Fig. 1 is a block diagram showing a configuration of an apparatus of an embodiment.
Fig. 2 is a block diagram showing a configuration of a processing unit.
Fig. 3 is a diagram showing a flow showing a concept of designing a primer in the related art.
Fig. 4 is a flowchart of a method of the embodiment.
Fig. 5 is a diagram for describing a graph coloring problem.
Figs. 6A to 6C are diagrams for describing a procedure of enumerating a maximal independent set of a graph example by ZDD.
Fig. 7 is a diagram for describing the procedure of enumerating the maximal independent set of the graph example by ZDD.
Fig. 8 is a diagram showing a flow showing a concept of a method of the embodiment.
Figs. 9A and 9B are diagrams for describing a graph color problem of a third embodiment.
Fig. 10 is a flowchart showing Modification Example 1.
Fig. 11 is a flowchart showing Modification Example 2.
Fig. 12 is a table showing 177 types of miRNAs.
Fig. 13 is a table showing a part of 684 pairs in which interaction is specified.

### DESCRIPTION OF THE PREFERRED EMBODIMENTS

Hereinafter, a method, an apparatus, and a program capable of measuring, in a case where a feature amount for predicting or discriminating a characteristic of a sample is selected and a measurer that measures the feature amount is designed, as many feature amounts as possible in one measurement while avoiding interaction between measurers, with reference to accompanying drawings.

### <<Apparatus of Embodiment>>

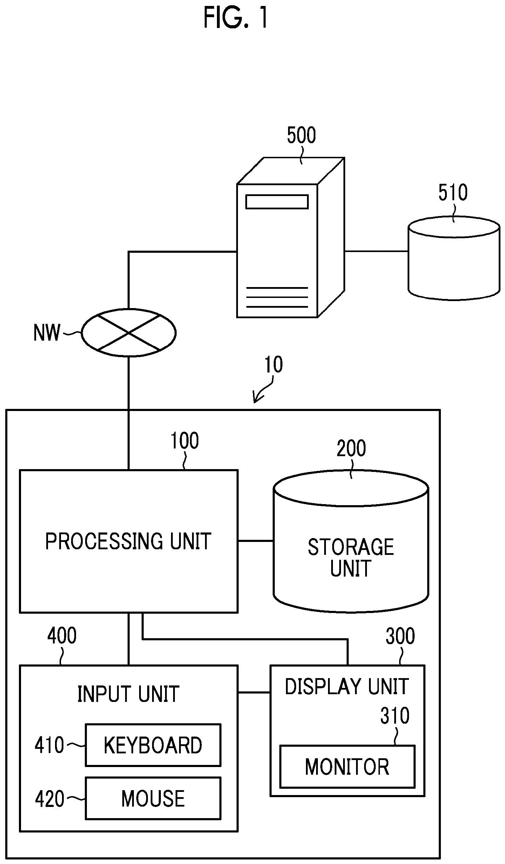

Fig. 1 is a block diagram showing a configuration of an apparatus 10 of an embodiment. The apparatus 10 is an apparatus that can measure as many feature amounts as possible while avoiding the interaction between the measurers, and can be realized by using a computer. As shown in Fig. 1, the apparatus 10 comprises a processing unit 100, a storage unit 200, a display unit 300, and an input unit 400, which are connected to each other to transmit and receive necessary information. Various installation forms can be employed for these components. For example, each component may be installed at one place (in one housing, in one room, or the like), or may be installed at a distant place and connected via a network. Further, the apparatus 10 may be connected to an external server 500 and an external database 510 via a network NW, such as the Internet. The apparatus 10 may acquire information, such as a program including an algorithm for realizing the processing of the embodiment, from the external server 500 and the external database 510, as necessary.

### <Configuration of Processing Unit>

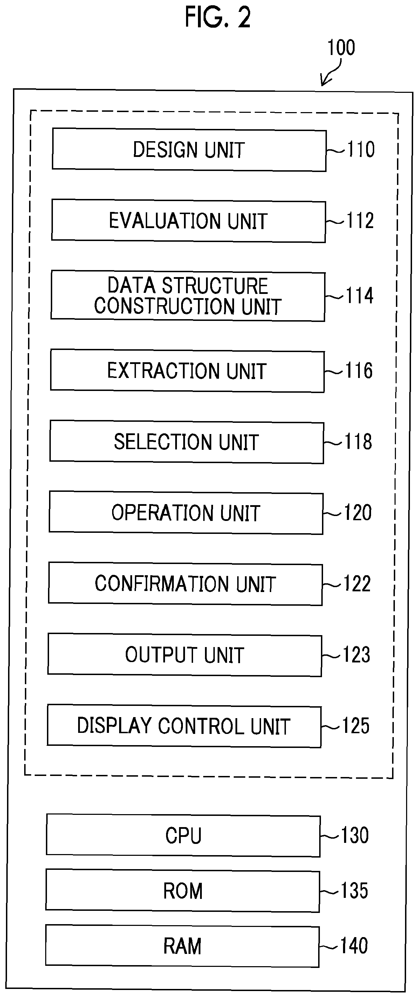

Hereinafter, an outline of each processing unit will be described. Fig. 2 is a block diagram showing a configuration of the processing unit 100. The processing unit 100 comprises a design unit 110, an evaluation unit 112, a data structure construction unit 114, an extraction unit 116, a selection unit 118, an operation unit 120, a confirmation unit 122, an output unit 123, a display control unit 125, a central processing unit (CPU) 130, a read only memory (ROM) 135, and a random access memory (RAM) 140.

The design unit 110 attempts to design the measurer for a feature amount candidate. The evaluation unit 112 evaluates the interaction between a plurality of measurers designed by the design unit 110. The data structure construction unit 114 associates, as a combination pair of the measurers that cannot be set at the same time, a combination of the measurers whose interaction evaluated by the evaluation unit 112 is problematic to construct a graph-like data structure. The extraction unit 116 selects, from the graph-like data structure constructed by the data structure construction unit 114, a portion corresponding to one or a plurality of independent sets to extract one or more feature amount set candidates that do not include the combination pair whose interaction is problematic. The selection unit 118 selects the feature amount set candidate or a subset of the feature amount set candidate as a feature amount set to be measured. The operation unit 120 performs an operation including the feature amount selection for each of the extracted feature amount set candidates. The confirmation unit 122 evaluates a result of the operation unit 120 and confirms the feature amount set to be measured based on the result of the evaluation.

The output unit 123 outputs the graph-like data structure constructed by the data structure construction unit 114, the feature amount set including the feature amount set candidate or the subset of the feature amount set candidate selected by the selection unit 118, or the like. The display control unit 125 controls the display of the acquired information and the processing result on a monitor 310. The processing by these functions is performed under the control of the CPU 130.

The functions of each part of the processing unit 100 described above can be realized by using various processors. The various processors include, for example, a CPU, which is a general-purpose processor that executes software (program) to realize various functions. The various processors described above also include a programmable logic device (PLD), which is a processor whose circuit configuration can be changed after manufacturing, such as a field programmable gate array (FPGA). Further, the various processors described above also include a dedicated electric circuit, which is a processor having a circuit configuration specifically designed to execute specific processing such as an application specific integrated circuit (ASIC).

The functions of each part may be realized by one processor or may be realized by combining a plurality of processors. A plurality of functions may be realized by one processor. As an example of configuring the plurality of functions by one processor, firstly, there is a form in which one processor is constituted of a combination of one or more CPUs and software and the processor realizes the plurality of functions, as represented by a computer such as a client or a server. Secondly, there is a form in which a processor that realizes functions of the entire system by one integrated circuit (IC) chip is used, as represented by a system on chip (SoC). As described above, a hardware structure for the various functions is constituted of using one or more of the various processors described above. Further, as the hardware structure of the various processors, more specifically, an electric circuit (circuitry) in which circuit elements such as semiconductor elements are combined may be used.

In a case where the above processor or electric circuit executes the software (program), a code readable by a processor (computer) of the executing software is stored in a non-transitory recording medium such as the ROM 135 (refer to Fig. 2) and the processor refers to the software. The software stored in the non-transitory recording medium includes a program for dividing a primer pair according to the present invention into a reaction container. The code may be recorded in various magneto-optical recording devices and a non-transitory recording medium such as a semiconductor memory instead of the ROM 135. In a case where processing using the software is performed, for example, the RAM 140 may be used as a transitory storage area or data stored in an electronically erasable and programmable read only memory (EEPROM) (not shown) may be referred to.

### <Configuration of Storage Unit>

The storage unit 200 is configured of a digital versatile disk (DVD), a hard disk, non-transitory recording media such as various semiconductor memories, and the like, and a control unit thereof.

### <Configuration of Display Unit and Input Unit>

As shown in Fig. 1, the display unit 300 comprises the monitor 310 (display device), and can display input information, information stored in the storage unit 200, a result of processing by the processing unit 100, and the like. The input unit 400 includes a keyboard 410 and a mouse 420 as an input device and/or a pointing device, and a user can perform an operation necessary for executing the processing according to the embodiment via these devices and a screen of the monitor 310.

### <Processing in Apparatus of Embodiment>

In the above-described apparatus 10, it is possible to measure, in a case where the feature amount for predicting or discriminating the characteristic of the sample is selected and the measurer that measures the feature amount is designed, as many feature amounts as possible while avoiding the interaction between the measurers, in response to a user's instruction via the input unit 400.

### <<Method of First Embodiment>>

Hereinafter, the method of the embodiment will be described. In the following, a case where the feature amount is a gene (for example, DNA methylation or micro ribonucleic acid (miRNA)) and the measurer is a PCR primer will be described as an example.

The feature amount may be one other than the gene, and the measurer is not limited to the primer and may be one corresponding to the feature amount. However, as the embodiment, in a case where a frequency of interaction between the measurers is high, in particular, in a case where a total number of feature amounts is desired to be increased, a high effect is obtained. Thus, the gene or the primer is a suitable case.

In machine learning or the like, a target sample to be classified or the like is prepared, a correct answer label is assigned to each sample, the feature amount is measured, and a classifier or the like is constructed based on a value of the feature amount. For example, a cancer type may be assigned to a biological tissue piece sample as the correct answer label. In each sample, a plurality of DNA methylation sites or miRNAs are measured, and a methylation degree, a count number, or the like is recorded for each of the DNA methylation sites or miRNAs. Based on the DNA methylation degree or the miRNA count number, a cancer classifier is constructed.

Here, one of factors that affect the performance of the classifier is the selection of the feature amount. In general, it is naturally desired that (1) in a case where the influence of the feature amount on the classification performance is unknown, many feature amounts are selected, or (2) in a case where the feature amount is known to contribute to the classification but there are many feature amounts having the same influence, a large number of feature amounts as many as possible are measured. However, there is also a case where the measurer needs to be designed for the feature amount and the design of the measurer is not always successful.

The design of the measurer for the feature amount will be described. For the selected DNA methylation site, the PCR primer that can measure the selected DNA methylation site is designed. The PCR primer (hereinafter simply referred to as primer) is a synthetic oligo DNA having a base length of several tens, and binds complementary to a vicinity of a target methylation site (hereinafter target) to sandwich the target methylation site in a pair.

However, the primer needs to specifically bind to a terminal of the target, that is, it is necessary to suppress a possibility of binding to DNA other than the target. Further, in general, the primer needs to control a reaction temperature, which is referred to as a Tm value, decided by the number, arrangement, or the like of bases to be in a predetermined range. Moreover, the complementarity between the primers also needs to be suppressed such that an amplifiction product referred to as a primer dimer generated by binding of any primers is not generated. In addition, other various conditions may be required for the primer, but many of the conditions are well known.

Here, the interaction between the measurers, for example, the avoidance of the primer dimer described above may be a problem. In the measurement of the DNA methylation site, in general, pretreatment referred to as bisulfite conversion is performed. That is, a chemical treatment is applied such that unmethylated cytosine (non-methylation cytosine) in DNA is converted into uracil while methylated cytosine is not converted. Accordingly, the methylation cytosine and the non-methylation cytosine can be distinguished from each other.

However, since it is not possible to decide in advance whether or not each site of the target is methylated, it is necessary to take into consideration, for example, that cytosine is not included in the primer. Thus, it may be difficult to design an appropriate primer for some targets, and the primer dimer may reduce a design success rate. Alternatively, in cancer estimation using miRNA of another case, since the miRNA is short and sequence homology with each other is high, it is difficult to avoid the primer dimer in designing, and thus the design success rate may be reduced.

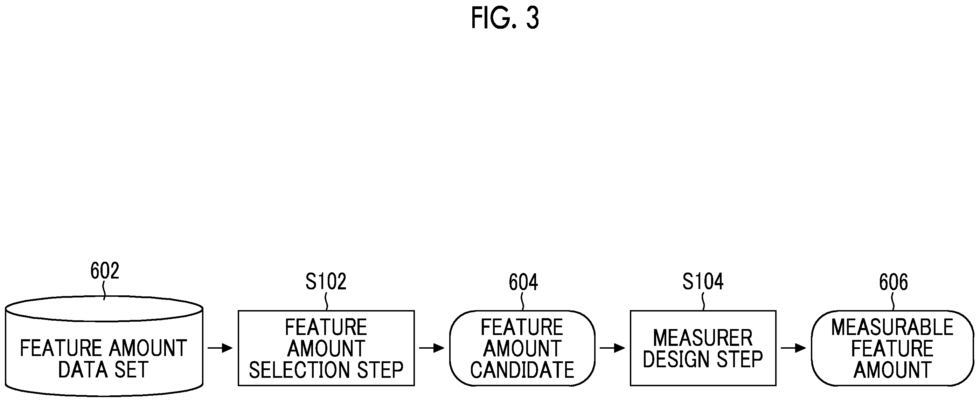

Fig. 3 shows a method in the related art in which the design success rate of the primer is not considered. As shown in Fig. 3, in the method in the related art, a feature amount candidate 604 is extracted from a feature amount data set 602 having a total feature amount as a population in a feature amount selection step (step S102), and a measurer design step (step S104) is performed on the feature amount candidate 604 to extract a measurable feature amount 606.

In this case, in a case where it is necessary to give up measuring the feature amount that is problematic with the primer dimer, there is a risk that the feature amount evaluated in the selection cannot be sufficiently measured. As a result of intensive studies, the inventors have reached the present invention, which is capable of measuring, in a case where the measurer that measures the feature amount is designed, as many feature amounts as possible while avoiding the interaction between the measurers.

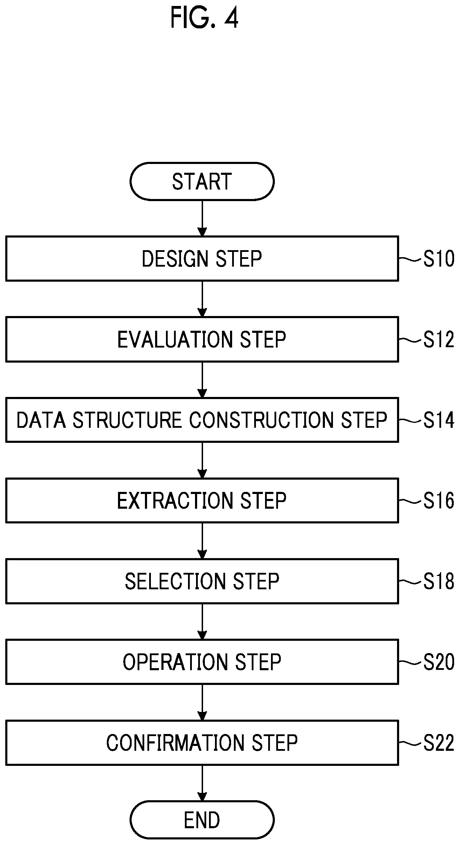

Fig. 4 is a flowchart showing the method of the embodiment. The method of the embodiment is a method of selecting one or a plurality of feature amounts that are used to predict or discriminate a characteristic of a sample and designing one or a plurality of measurers that measure the feature amount, the method comprising a design step (step S10) of attempting to design the measurer for a feature amount candidate, an evaluation step (step S12) of evaluating interaction between the designed plurality of measurers, a data structure construction step (step S14) of associating, as a combination pair of the measurers that are unable to be set at the same time, a combination of the measurers whose interaction evaluated in the evaluation step is problematic to construct a graph-like data structure, an extraction step (step S16) of selecting a portion corresponding to one or a plurality of independent sets from the graph-like data structure to extract one or more feature amount set candidates that do not include the combination pair whose interaction is problematic, and a selection step (step S18) of selecting, as a feature amount set to be measured, the feature amount set candidate or a subset of the feature amount set candidate. An operation step (step S20) and a confirmation step (step S22) will be described in a second embodiment. The operation step (step S20) and the confirmation step (step S22) are executed as necessary.

Hereinafter, each step will be described. In the following, a specific embodiment in a case where the measurer is the primer will be described.

### <Design Step (Step S10)>

The design unit 110 of the apparatus 10 performs the design step (step S10). The design step is a step of attempting to design the measurer for the feature amount candidate.

First, in order to amplify each target nucleic acid of a plurality of target nucleic acids that are feature amount candidates, primers having an inverted complementary sequence are designed at both ends of each target nucleic acid (base sequence). The setting can be made under various conditions depending on a purpose or the like.

The primer is the synthetic oligo DNA having the base length of several tens, and binds complementary to a vicinity of a target nucleic acid, for example, a DNA methylation site to sandwich the DNA methylation site in a pair.

The target nucleic acid is not particularly limited to DNA, ribonucleic acid (RNA), and the like. However, in a case where a non-specific reaction is likely to be induced, the target nucleic acid is particularly effective.

Here, the plurality of target nucleic acids that are the feature amount candidates can enumerate the gene to be measured and select the target nucleic acid (base sequence) to be amplified. Any number of target nucleic acids and any target can be selected as the target nucleic acid. For example, in a case of miRNA or the like, a type of the primer is not limited, such as distribution of a stem loop RT primer. A total number of target nucleic acids is preferably 50 or more and more preferably 100 or more.

The feature amount candidate is, for example, selected from a feature amount data set of a sample group in which values of a plurality of feature amounts are recorded. Further, by performing a feature amount data set input step, the values of the plurality of feature amounts are input to the feature amount data set. The feature amount data set is configured of a known sample group that belongs to a given class as a target and a feature amount group of the known sample group. It is assumed that a learning data set is provided and each sample is assigned with a value of a plurality of common feature amounts (for example, DNA methylation sites) and one correct answer class label (for example, cancer or non-cancer and tissue classification).

Further, input sample data may be divided into learning data and test data. The cancer type is assigned as the correct answer label to each sample of the learning data. Further, each sample has the plurality of DNA methylation sites, and the methylation degree is assigned to each of the DNA methylation sites. This methylation degree can be, for example, a value measured by a comprehensive measurement means such as a microarray.

The feature amount candidate can be selected by transferring or reading out a part of the feature amount from the feature amount data set.

### <Evaluation Step (Step S12)>

The evaluation unit 112 of the apparatus 10 performs the evaluation step (step S12). The evaluation step is a step of evaluating the interaction between the designed plurality of measurers.

In the evaluation step, dimer formability between the primer pair is calculated as the interaction between the plurality of measurers. The dimer between the primer pair (primer dimer) can be evaluated, for example, based on base homology between the primers. The base homology can be calculated by a local sequence alignment algorithm or the like in which a 3'-terminal sequence side is fixed. The evaluation can be performed by checking the number of matches (so-called "Match"), the number of mismatches ("Mismatch"), and insertion/deletion (In/Del) based on the base homology.

However, in the embodiment, the primer dimer may be evaluated by various methods without being limited to a specific determination method and a threshold value.

The interaction is not limited to the primer dimer, and includes interaction that may induce non-specific amplification.

### <Data Structure Construction Step (Step S14)>

The data structure construction unit 114 of the apparatus 10 performs the data structure construction step (step S14). In the data structure construction step, the combination of the measurers whose interaction evaluated in the evaluation step is problematic is associated as the combination pair of the measurers that are unable to be set at the same time to construct a graph-like data structure.

The data structure construction unit 114 generates a non-specific graph or a data structure corresponding to the non-specific graph (referred to as graph-like data structure) based on the calculated dimer formability. Specifically, the primer pair is associated with a node of the graph, and presence or absence of the primer dimer between the primer pair is associated with (presence or absence of) an edge of the graph to generate a primer non-specific graph. The number of nodes in the graph is the number of primer pairs, that is, the number of target nucleic acids, but the number of edges depends on a calculation result.

Various types of pretreatment may be performed on the graph. For example, a so-called graph division of dividing the graph into a plurality of connected graphs may be performed. With the graph division in this manner, it is only necessary to obtain an "independent set" described below for each divided graph and merge the obtained "independent sets". Therefore, the processing can be performed efficiently.

Further, a gene site group that is particularly dense may be, for example, subjected to sorting of so-called representative gene sites, which are represented by only one type (or a small number of types). With the sorting of the representative gene sites, it is possible to avoid unnecessarily complicated processing. For example, in a case where 16 nucleic acids of target nucleic acids X1, X2, ..., and X16 form a clique (partial graph that is a complete graph) in a graph and it is sufficient to measure any one of X1, X2, ..., or X16, only one type (or a small number of types) of X1, X2, ..., or X16 may be a measurement target, and others may be excluded from the measurement target. It should be noted that, for simplicity, the description has been made of the clique, but the same applies even in a case where a high-density partial graph similar to the clique can be extracted.

### <Extraction Step (Step S16)>

The extraction unit 116 of the apparatus 10 performs the extraction step (step S16). In the extraction step, with the selection of the portion corresponding to one or the plurality of independent sets from the graph-like data structure, one or more feature amount set candidates that do not include the combination pair whose interaction is problematic are extracted.

In the extraction step, one or more or the maximum number of the independent sets are extracted from the graph-like data structure constructed in the data structure construction step (step S14) as one of the feature amount set candidates.

In the extraction step, the "independent set" is specified from the graph. The "independent set" is a set of vertices in which no adjacent pair of vertices is included at all, and the "independent set" does not include a possibility of forming the primer dimer. As a result, it is not necessary to consider the interaction in designing the measurer. Therefore, in the embodiment, the "independent set" is selected as a population candidate for the feature amount selection.

For example, each "color class" in a case where a graph coloring problem is solved is the "independent set". Here, the graph coloring problem is a solution that allocates colors to vertices such that no adjacent vertices have the same color to minimize the number of colors. Among these, a vertex group colored with the same color is referred to as the "color class".

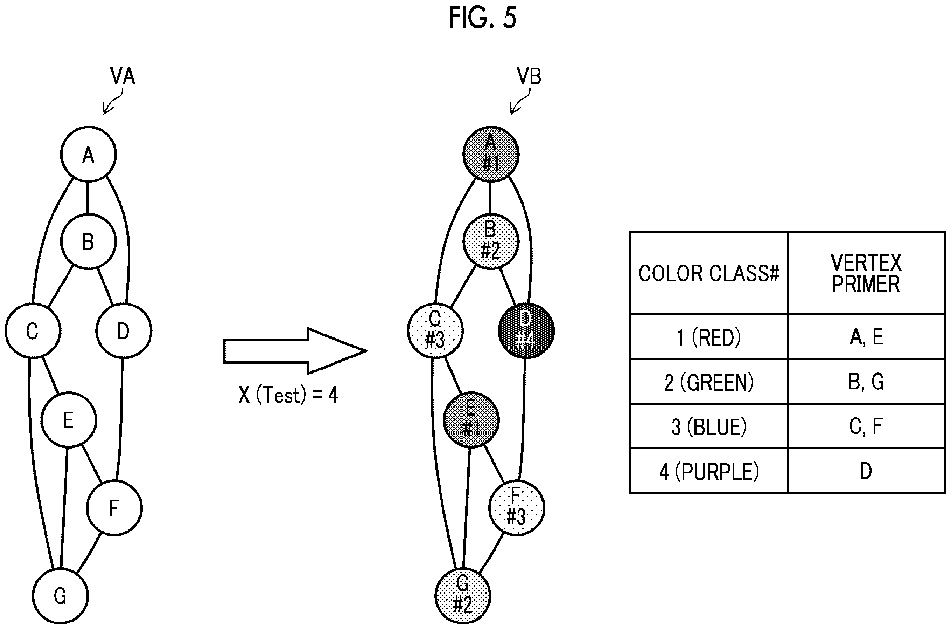

Fig. 5 is a diagram for describing the graph coloring problem. A graph VA is a diagram showing an example of the primer non-specific graph (graph). The graph VA is the primer non-specific graph created for seven primer pairs, and each primer pair is indicated by A to G. Each primer pair is a vertex (node), and the primer pairs evaluated, in the evaluation step, that the primers constituting the primer pair are the primer dimers are connected by a side (edge or link). The number of vertices in the graph is the number of primer pairs, that is, the number of target nucleic acids. Further, the number of sides depends on the calculation result in the evaluation step, and is the number determined to be the primer dimer.

The solution for the graph coloring problem is applied to the graph VA, and the vertices are colored with a plurality of conceptual colors such that the vertices adjacent to each other via the sides have different colors. A graph VB in Fig. 5 is a graph in which the primer non-specific graph shown in the graph VA is colored. As shown in the graph VB, with coloring of the primer pair (A, E) in red, the primer pair (B, G) in blue, the primer pair (C, F) in green, and the primer pair (D) in purple, it is possible to prevent any adjacent vertices from being the same color. Each of the primer pair (A, E), the primer pair (B, G), the primer pair (C, F), and the primer pair (D) is the color class and the independent set. That is, the graph coloring problem is solved, and then the color class having the largest size may be selected.

For example, a Welsh-Powell method and the like are known as a solution for a heuristic graph coloring problem, and the coloring can be performed using such a method.

It should be noted that, instead of a mere "independent set", it is further desirable to obtain a "maximal independent set" or a "largest independent set". Here, the "maximal independent set" is the independent set that is no longer the independent set even in a case where any vertex is added, and the "largest independent set" is the independent set having a maximum size. Since all the subsets of the "maximal independent set" are the "independent sets" and the "largest independent set" is also the "maximal independent set", it is more suitable in a case where a large number of feature amounts are desired to be measured.

### <Selection Step (Step S18)>

The selection unit 118 of the apparatus 10 performs the selection step (step S18). In the selection step, the feature amount set candidate or the subset of the feature amount set candidate is selected as the feature amount set to be measured.

In the selection step, the feature amount set candidate extracted in the extraction step can be used as the feature amount set to be measured. Further, in the selection step, the subset of the feature amount set candidate can be used as the feature amount set. In the subset of the feature amount set candidate, a part of the feature amount set candidate can be selected instead of all of the feature amount set candidates.

In the selection step, it is possible to select the feature amount set that allows the design of the measurer while avoiding the interaction between the measurers by the independent set. Further, in a case where the maximal independent set is selected, it is preferable since there is selection where a new feature amount (or new measurer) cannot be further added. In a case where the largest independent set is selected, the maximum number of feature amount sets can be selected, which makes it possible to measure a large number of feature amounts in one measurement.

Next, the graph-like data structure will be described including another aspect. As described above, the description is made here as the "graph coloring problem" in the most understandable manner, but many NP-complete problems to which the graph coloring problem belongs are proved to be equivalent to each other. Thus, it is possible to solve the graph coloring problem as another problem in a modified manner, that is, to apply a data structure other than the graph data structure or an algorithm. For example, a complementary set of the independent set is a vertex cover. Therefore, regardless of which side of the graph is taken, at least one of end points is included in the vertex cover. Here, it is known that a vertex cover problem can be transformed into a partial sum problem. Therefore, the vertex cover problem can be transformed into a problem in which the graph does not appear clearly at first glance, such as the partial sum problem, and a solution to such a transformed problem or the problem can be considered. Further, although the equivalent transformation is described above, for example, the problem may be transformed into another problem that satisfies a sufficient condition or may be transformed into another problem that can obtain an approximate solution. In the embodiment, these are collectively referred to as the "graph-like data structure".

Such a transformation can be made in various ways, and is useful in a case where various algorithms, software modules, or the like, which are excellent or familiar in the related art, are desired to be reused.

Further, a search using ZDD as a staining method can also be used.

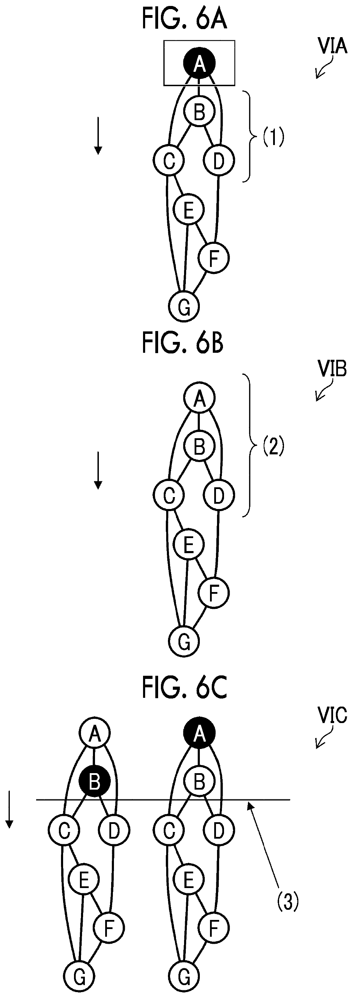

Further, as a coloring step, the search using ZDD can be performed. Specifically, for example, the above-described coloring problem can be divided into "enumeration of maximal independent set (MIS)" and "enumeration of entire graph covering by MIS" to construct corresponding ZDDs. Figs. 6A to 7 are diagrams for describing a procedure of enumerating the maximal independent sets of the graph example by ZDD. First, for the created primer non-specific graph, "pruning" and "section sharing" are performed to reduce a pattern to be identified. The "pruning" refers to processing of selecting a certain vertex and identifying whether or not it is confirmed to be unsuitable without considering the presence or absence of remaining selection to reduce the pattern to be identified. Further, the "section sharing" refers to processing of aggregating a pattern group such that, in a case where a subsequent selection matches in a different selection pattern, a branch thereof is the same to reduce the pattern to be identified. The vertices are selected in order in a direction indicated by an arrow in Figs. 6A to 6C.

A graph VIA of Fig. 6A is a diagram for describing a pruning condition (1), and a graph VIB of Fig. 6B is a diagram for describing a pruning condition (2). In the pruning condition (1), the "pruning" is performed at a point in time where a vertex adjacent to a selected vertex is selected. In the coloring step, the vertices adjacent to each other via the sides are colored to be different colors. Therefore, at a point in time where the vertices B, C, and D adjacent to the selected vertex A are selected, the combination by the selection is unsuitable, and thus it is not necessary to consider the subsequent selection. Further, in the pruning condition (2), the "pruning" is performed at a point in time where an addable (selectable) vertex is not selected. The independent set obtained in a case where all the vertices A to D are not selected is still the independent set even in a case where the vertex A is added. Therefore, the independent set is no longer the "maximal" independent set.

A graph VIC of Fig. 6C is a diagram for describing a section sharing condition (3). The section sharing is performed at a point in time where the set of the selected vertex and the set of the adjacent vertex match each other. That is, in a case where the selected vertex A and the adjacent vertex (B) thereof are compared, the adjacent vertices of the vertex A are the vertices B, C, and D, and the adjacent vertices of the vertex B are the vertices A, C, and D. Therefore, since the subsequent selection is common in a case where the vertex A is selected and a case where the vertex B is selected, the branches of ZDD are joined to share the subsequent selection processing. Accordingly, in a case where any one of the vertices A and B is selected, overlap processing does not have to be performed individually.

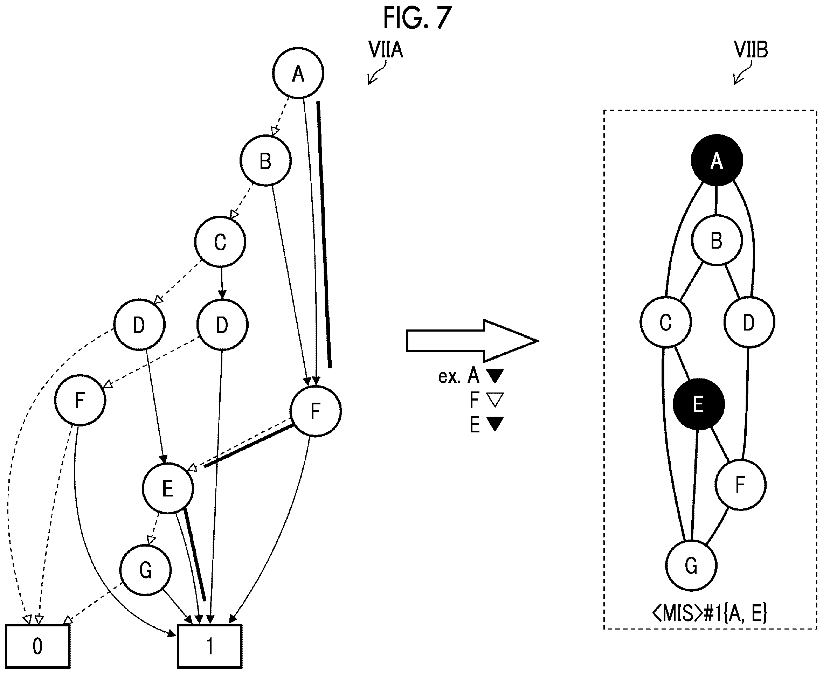

For example, in Fig. 7, since the selection of A (A▼) and the non-selection of A and the selection of B (A∇B▼) join the same F, in a case where any one of the vertices A and B is selected, the overlap processing does not have to be performed individually.

In a case where the maximal independent set, that is, all the candidates for the color class are enumerated under these conditions, a ZDD expression of the maximal independent set enumeration as a graph VIIA shown in Fig. 7 is obtained. With selection of a vertex in a case of being indicated by an arrow ▼ using this graph VIIA and non-selection of a vertex in a case of being indicated by an arrow ∇, a selectable combination is set to 1 and a non-selectable combination is set to 0. That is, the combination set to be 1 is a case where any one of the vertices adjacent to each other via the sides is selected and any one of the vertex that determines whether the selection is possible and the vertex adjacent to the vertex via the sides is selected. For example, in the graph VIIA, with selection of A (A▼), non-selection of F (VF), and selection of E (E▼), it is possible to create a graph showing an example of extracting the maximal independent set shown in a graph VIIB.

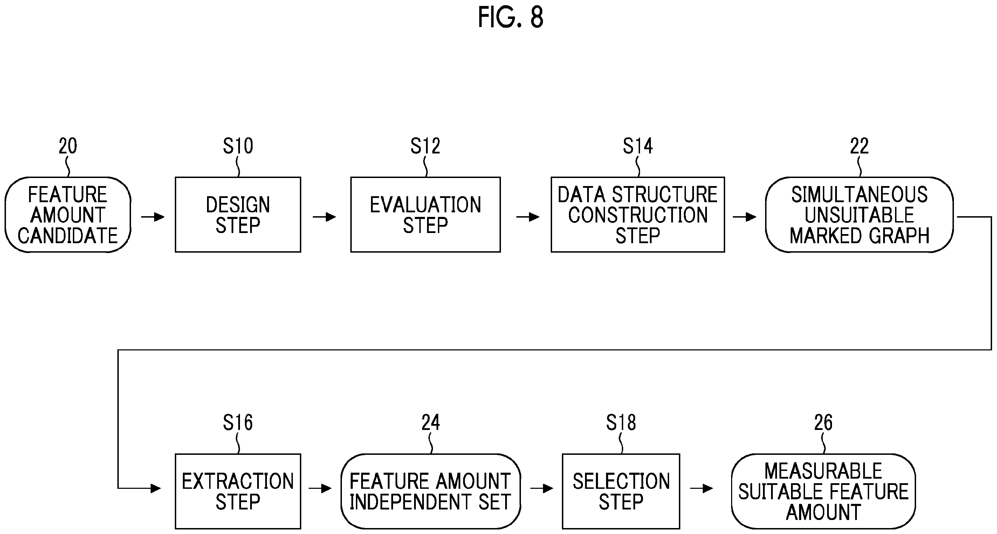

Fig. 8 is a diagram showing a flow showing a concept of the method according to the embodiment. As shown in Fig. 8, in the design step (step S10), the design of the measurer is attempted for a feature amount candidate 20 selected from the feature amount data set (not shown). Next, the interaction between the measurers is evaluated for the designed measurer in the evaluation step (step S12). In the data structure construction step (step S14), with the association as the combination pair of the measurers that cannot be set at the same time, the graph-like data structure as shown in Fig. 5, for example, a simultaneous unsuitable marked graph 22 is constructed. In the extraction step (step S16), with the selection of the portion corresponding to one or the plurality of independent sets from the simultaneous unsuitable marked graph 22 which is the graph-like data structure, the feature amount set candidate that does not include the combination pair whose interaction is problematic, for example, a feature amount independent set 24 is selected. In the selection step (step S18), the feature amount set candidate or the subset of the feature amount set candidate can be selected as the feature amount set to be measured, for example, a measurable suitable feature amount 26.

Next, preferred aspects of the embodiment will be described.

### <<Method of Second Embodiment>>

Next, a method of the second embodiment will be described. In the second embodiment, in a case where one or the plurality of maximal independent sets are selected, among the plurality of independent sets, to extract one or more feature amount set candidates in the extraction step (step S16), the operation step (step S20) of performing the operation including the feature amount selection for each of the selected feature amount set candidates and the confirmation step (step S22) of evaluating the result of the operation step (step S20) and confirming the feature amount set to be measured based on the result of the evaluation are executed after the selection step (step S18), as shown in a flowchart of Fig. 4. That is, the plurality of maximal independent sets may be enumerated, the feature amount set may be selected from each of the plurality of maximal independent sets and then may be evaluated, and the feature amount set having the best evaluation result may be confirmed.

The selection unit 118 of the apparatus 10 performs the selection step (step S18) of the second embodiment. In the selection step (step S18), the plurality of maximal independent sets are selected as the feature amount set candidates, instead of simply selecting the maximum number of feature amount sets, that is, the largest independent set.

The operation unit 120 of the apparatus 10 performs the operation step (step S20), and the confirmation unit 122 performs the confirmation step (step S22). In the operation step (step S20) and the confirmation step (step S22), the optimum feature amount set may be confirmed by another evaluation method. For example, for each of the feature amount set candidates or the subset thereof, machine learning performance based on the feature amount, such as a class classification, can be evaluated to perform the confirmation as the best feature amount set.

Among all the combinations of the feature amounts, the evaluation can be made from the candidate for which the measurer can be designed and the number of measurers is large. Therefore, it is expected that an excellent feature amount set can be efficiently selected.

For example, in the second embodiment, in the operation step (step S20), for example, #1 (red) (A, E), #2 (green) (B, G), and #3 (blue) (C, F) are selected as the feature amount set candidates shown in Fig. 5. In addition to #1 to #3, #4 (purple) (D) may be selected as the candidate. Further, since the size is small, #4 (purple) (D) may not be selected as the candidate. After #1 to #3 are selected, each feature amount set is evaluated. Examples of the method of evaluating the feature amount set include the number of selectable feature amounts and the performance of the classifier depending on the feature amount. However, the present disclosure is not limited thereto, and various methods of evaluating the feature amount set can be applied.

In the confirmation step (step S22), the result of the operation step (step S20) may be evaluated, and for example, the feature amount set having the highest evaluation result may be confirmed.

### <<Method of Third Embodiment>>

Next, a method of a third embodiment will be described. In a case where the measurer is designed, the plurality of measurers may be designed for one feature amount, and the interaction with other measurers may be different depending on the measurer. For example, in the primer design for gene measurement, a base sequence may be different depending on a primer position, and thus the primer dimer formability may be different.

In this case, the selection of the primer for a certain gene may affect the selection of the primer for another gene by the interaction. In such a case, it is necessary to consider a combination of the feature amounts to be measured and the measurers for each feature amount. Thus, it is more difficult to set an appropriate feature amount and measurer. Therefore, as a result of intensive studies on the setting of the feature amount and the measurer, the inventors have reached the proposal of the following embodiments.

The design unit 110 of the apparatus 10 performs the design step (step S10) of the third embodiment. In the third embodiment, in the step (step S10) of attempting to design the measurer, the design of the plurality of the measurers is attempted for each of at least one or more feature amount candidates.

As described above, in a case where the plurality of the measurers can be designed for one feature amount in designing the measurer, the design of the plurality of measurers is attempted.

The data structure construction unit 114 of the apparatus 10 performs the data structure construction step (step S14). In the data structure construction step, all the plurality of measurers are considered, and any measurer pair for the same feature amount candidate is associated as the combination pair that cannot be set at the same time.

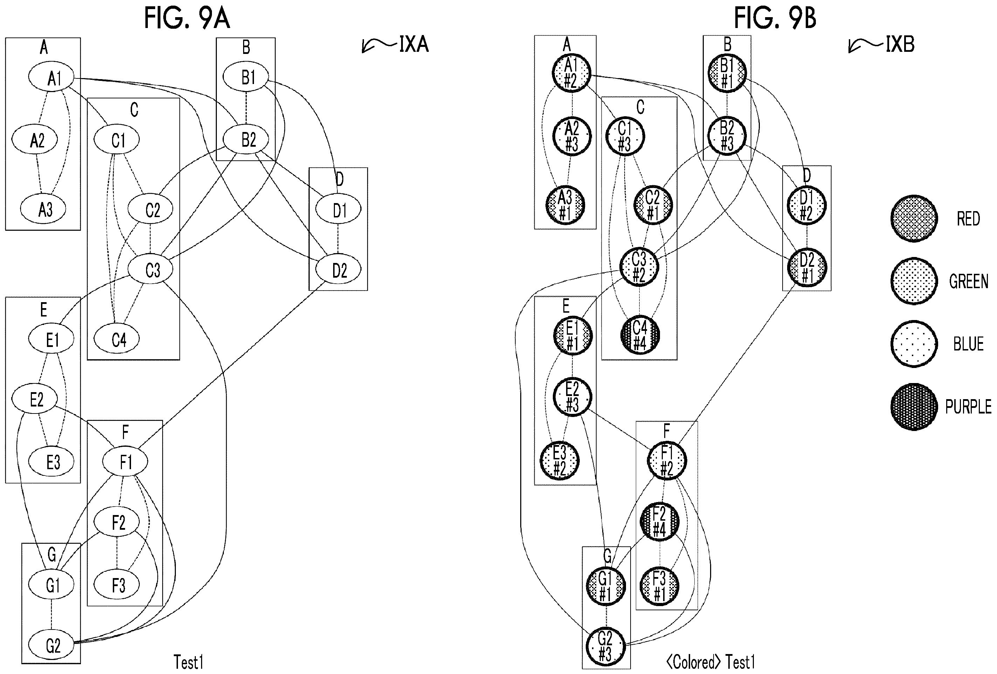

Figs. 9A and 9B are diagrams for describing a graph color problem of the third embodiment. A graph IXA is a diagram showing an example of the primer non-specific graph (graph). For example, the graph IXA is created by designing the plurality of primers for at least one gene in the creation of the non-specific graph, checking the dimer formability of all the designed primers to generate a graph side (edge) in the generation of the non-specific graph, and further virtually connecting all the primers for the same gene by the side. In the graph IXA, each square frame line indicates a primer group for the same gene. For example, (A1, A2, A3) in a box A indicates three types of measurers for a gene A. Similarly, (B1, B2), (D1, D2), and (G1, G2) in boxes B, D, and G indicate two types of measurers for genes B, D, and G. Similarly, (E1, E2, E3) and (F1, F2, F3) in boxes E and F indicate three types of the measurers for genes E and F. Further, (C1, C2, C3, C4) in a box C indicates four types of the measurers for a gene C. In the graph IXA, the broken line is drawn as the side between the primers for the same gene in order to facilitate understanding, but the solid line can be regarded as the same as other solid lines in the operation.

A graph IXB of Fig. 9B is a graph in which the pretreatment shown in the graph IXA is performed and then the independent set is similarly selected in subsequent processing. As shown in the graph IXB, since the primers for the same gene are connected to each other, only one primer for a certain gene is selected in any independent set. That is, the boxes A to G do not include two or more of a certain color. That is, the colors of the respective measurers are different in the boxes A to G. For example, in a case where the red color (A3, B1, C2, D2, E1, F3, G1) is focused on, this is a set example in which only one primer is selected for the same gene, then the red color is arranged in all the boxes A to G, and thus all the (= maximum number of) genes can be measured. That is, it is possible to select all the feature amounts while avoiding the interaction between the measurers. For example, A2 may be selected instead of A3, which is an example and is not particularly limited.

On the other hand, for example, in a case where the measurers for a certain gene are collectively made into a single node and a method is employed in which the sides are drawn in a case where at least any one of the measurers interacts, there is the graph as shown in Fig. 5. In the graph shown in Fig. 5, it is possible to avoid the interaction between the measurers, but the number of genes that can be measured may be reduced.

In the third embodiment, it is possible to select the combination pair of the appropriate feature amount and the measurers that may be present in plural for the feature amount.

### <Preferred Modification Example>

Next, the "design in consideration of measurer mutual relationship", which are the embodiments described in the first embodiment, the second embodiment, and the third embodiment, can be incorporated into, for example, a method of selecting a measurable suitable feature amount in which a measurable feature amount is selectable, in a case where the feature amount selection and the measurer design are required, with coordination between the feature amount selection and the measurer design. Hereinafter, preferred modification examples will be described.

### <Modification Example 1>

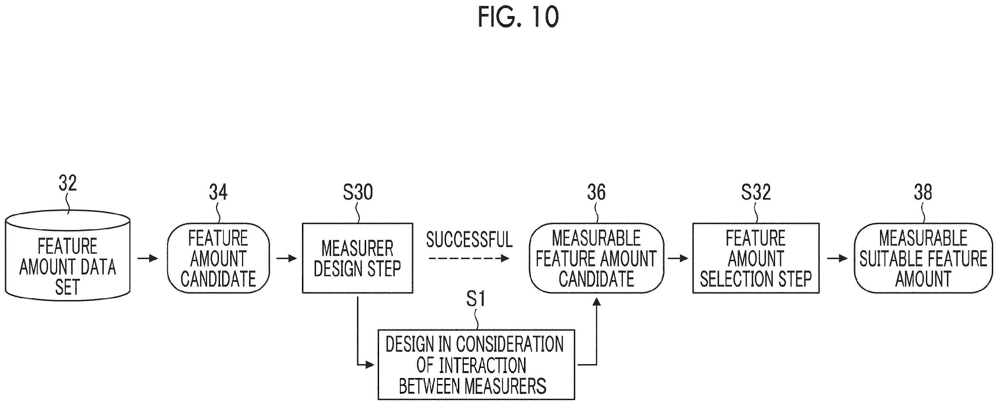

Fig. 10 is a flowchart showing a preferred modification example 1. In Modification Example 1, a feature amount candidate 34 is extracted from a feature amount data set 32 through a feature amount candidate extraction step. Since the feature amount data set 32 includes information, such as a sample name and a class label, other than the feature amount, the feature amount is extracted as the feature amount candidate 34 from the feature amount data set 32. As a method of extracting the feature amount candidate 34, a method of transcribing or reading out the feature amount portion from the feature amount data set 32 can be used.

In the measurer design step (step S30), the measurer is designed for the feature amount candidate 34. In the measurer design step (step S30), the measurer is designed for the feature amount candidate 34, and the feature amount for which the design of the measurer has succeeded is extracted as a measurable feature amount candidate 36. The measurer design step (step S30) can be performed in the same manner as in the design step (step S10). Next, in the feature amount selection step (step S32), the feature amount is selected from the measurable feature amount candidate 36 by a desired number to be set as a measurable suitable feature amount 38. In the feature amount selection step (step S32), the characteristic of the sample can be predicted or discriminated, and the best feature amount can be selected in a range of the feature amount for which the design of the measurer is successful. In the measurer design step (step S30), the measurer is designed for all of the feature amount candidates 34. Therefore, the measurer design step (step S30) is particularly effective in a case where a measurer design cost is low. Further, in the present invention, examples of the cost include a required time, a memory consumption, and an actual cost. However, the cost is not limited thereto. The same also applies to the following embodiments.

The design step (step S1) of the embodiment in consideration of the measurer mutual relationship can be applied in combination to the measurer design step (step S30) and the extraction of the measurable feature amount candidate 36 of Modification Example 1 described above. For example, with the application of the design step (step S1) in consideration of the measurer mutual relationship to the feature amount candidate 34 of Modification Example 1, it is possible to extract the measurable feature amount candidate 36 in which the interaction between the measurers is considered. Next, in the feature amount selection step (step S32), the measurable suitable feature amount 38 is selected from the measurable feature amount candidate 36 in which the interaction between the measurers is considered.

The measurable suitable feature amount 38 can be selected by a known method. A so-called filter method, wrapper method, or embedded method may be used. Specifically, for example, any of various methods introduced in papers, such as "A review of feature selection techniques in bioinformatics" (Yvan Saeys, 2007: Bioinformatics), may be used. In particular, the effect of the present invention is high in a case where a method in which a combination of feature amounts is effective is applied. For example, the Sequential Backward Elimination approach of the wrapper method is a method that first selects all feature amounts and then removes the feature amounts with the least degree of deterioration in class classification performance one by one and in which a combination of the feature amounts is effective. As another example, the method described in WO2021/161901A, which is an application by the applicant, may be applied.

### <Modification Example 2>

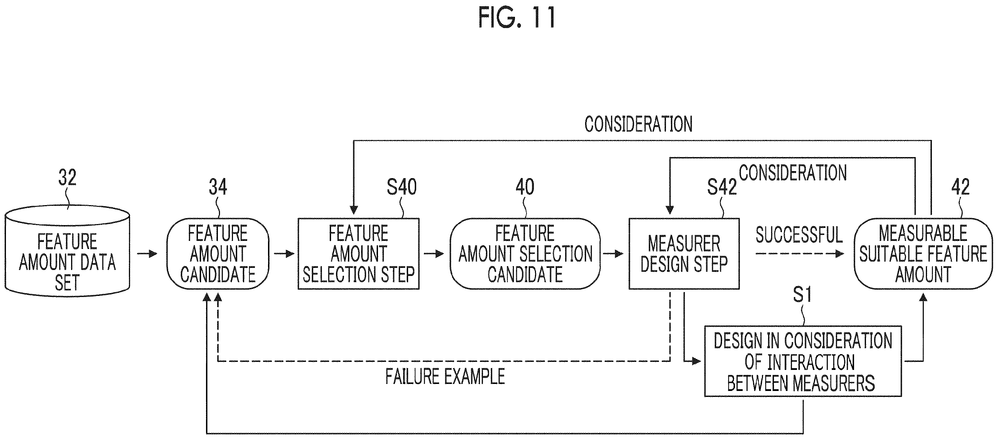

Fig. 11 is a flowchart showing a preferred Modification Example 2. In Modification Example 2, the feature amount candidate 34 is extracted from the feature amount data set 32 through the feature amount candidate extraction step as in Modification Example 1. In the feature amount selection step (step S40), the feature amount for predicting or discriminating the characteristic of the sample is selected from the feature amount candidate 34 as a feature amount selection candidate 40. The feature amount selection step (step S40) can be performed by the same method as in the feature amount selection step (step S32) of Modification Example 1. In the measurer design step (step S42), the measurer is designed for the feature amount selection candidate 40. In the measurer design step (step S42), the measurer is designed for the feature amount selection candidate 40, and the feature amount for which the design of the measurer has succeeded is extracted as a measurable suitable feature amount 42. The measurer design step (step S42) can be performed by the same method as in the measurer design step (step S30) of Modification Example 1.

In Modification Example 2, one or a small number of feature amounts are temporarily selected, and the design of the measurer is attempted for the temporarily selected feature amount selection candidates 40. In a case where the design of the measurer is successful, the selection of the feature amount is confirmed and added to the measurable suitable feature amount 42. In a case where the design of the measurer fails, the feature amount thereof is removed from the original feature amount candidate 34. This is repeated until the desired number of measurable suitable feature amounts 42 are selected.

All the designs of the measurer for the measurable suitable feature amount 42 to be output are successful. Moreover, in the feature amount selection step (step S40), a next feature amount selection candidate can be selected in consideration of an already confirmed measurable suitable feature amount 42. Similarly, in the measurer design step (step S42), the measurer for a next feature amount selection candidate 40 can be designed in consideration of the already confirmed measurable suitable feature amount 42.

For example, in a case of a feature amount selection method of evaluating a combination of the feature amounts to derive some evaluation value, one or a small number of feature amounts with a high selection priority, which improve the evaluation value the most in a case of being added to the current measurable suitable feature amount 42, may be specified by a greedy search and may be used as a next temporarily selected feature amount. The evaluation value may be decided by the feature amount selection method. For example, in a case of a wrapper method, the class classification performance may be used.

Further, the success or failure of the design of the measurer may be determined individually for each feature amount selection candidate 40, for example, from specificity of the primer sequence, the Tm value, or a CG ratio, may be determined in consideration of the interaction with the set of the designed measurers, for example, the dimer formation between the primers, or may be determined by combining both. Although the "design" is described here, the "design" may include, for example, deciding the base sequence of the primer, or since there may be problems that cannot be solved by prior studies, the "design" may include checking that the feature amount of the sample can be measured by an experiment. In this embodiment, since the feature amounts may be selected one by one or in small numbers and the measurer may be designed, the present invention can be flexibly applied to various cases.

The design step (step S1) of the embodiment in consideration of the measurer mutual relationship can be applied in combination to the measurer design step (step S42) and the extraction of the measurable suitable feature amount 42 of Modification Example 2 described above. For example, with the application of the design step (step S1) in consideration of the measurer mutual relationship to the feature amount selection candidate 40 of Modification Example 2, it is possible to extract the measurable suitable feature amount 42 in which the interaction between the measurers is considered.

### [Other Application Examples]

The aspect in which the feature amount is a gene, the gene is the information of the DNA methylation site, and the measurer is the primer which is gene measurement means has been described above. However, the present invention is not limited thereto. A biomarker, such as DNA mutation, mRNA, miRNA, a protein, or a metabolite can be used as the gene. Further, a probe can be used as the measurer.

Furthermore, the present invention can also be applied to the following problems.
(1) Design of diagnosis: in a problem for the purpose of detecting a disease, medical examination items are used as the feature amounts, and examination means that can be implemented in any diagnosis form, such as a health checkup, among the feature amounts is used as the measurer. For example, it may be determined whether or not the examination means (measurer) can be designed according to a required examination skill level, an examination cost, an examination time, and the like.
(2) Consideration of privacy and the like: in a problem for the purpose of estimating personal preference and the like, a questionnaire and a personal attribute are used as the feature amounts, and, for example, acquisition means that can be implemented by privacy and related regulations among the feature amount is used as the measurer. For example, it may be determined whether or not the acquisition means (measurer) can be designed according to a required regulation level, an acquisition cost, an acquisition time, the response rate of the questionnaire, and the like.
(3) Drone imaging: in a problem of determining the state of a building and the like, captured images are used as the feature amounts, and an image that can be captured by, for example, a drone among the feature amounts is used as the measurer. For example, it may be determined whether or not imaging means (measurer) can be designed according to the number of consecutively captured images, the difficulty of imaging, regulations, and the like.

Since each of the above aspects is embodied in an executable form, it is clear that the present invention can be similarly applied to each problem or a wide general problem only by replacing the specific representations of the feature amount and the measurer. Further, whether or not the measurer can be designed may be determined not only based on technical difficulty but also based on regulations, financial or time costs, a success rate of acquisition, and the like. In any case, the selected feature amount can be flexibly configured by an examination format, privacy regulations, drone performance, related regulations, or the like based on a certain master data set, or seamless development is possible in a case where the measurer is switched by a study on practical use after a feasibility study.

### Examples

Hereinafter, the present invention will be described in more detail with reference to examples of the present invention.

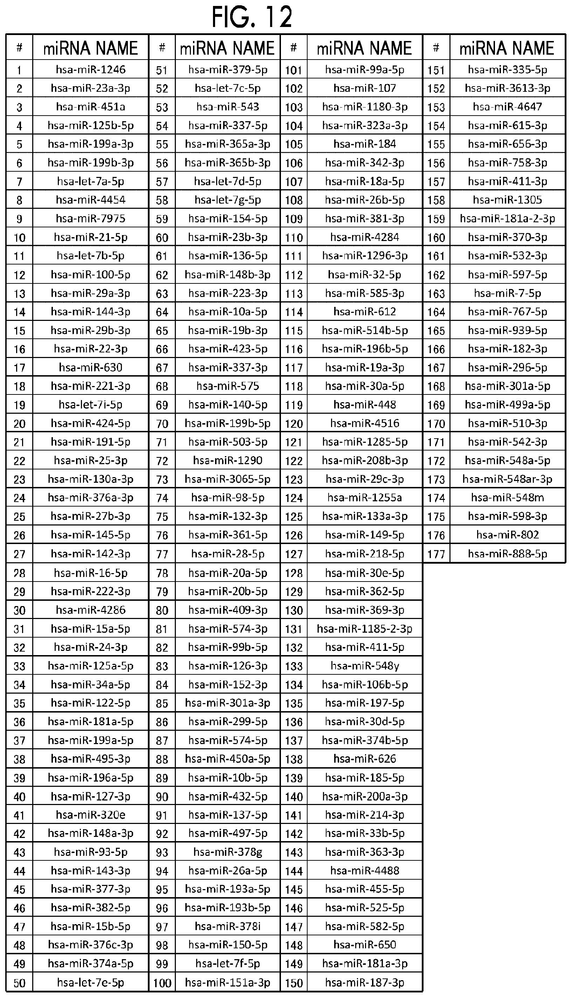

The inventors study the maximization of the number of genes that can be simultaneously measured using 177 types of miRNAs [Ferguson, Scott W., et al., 2018] that are specifically highly expressed in MSC. Fig. 12 is a table showing 177 types of miRNAs.

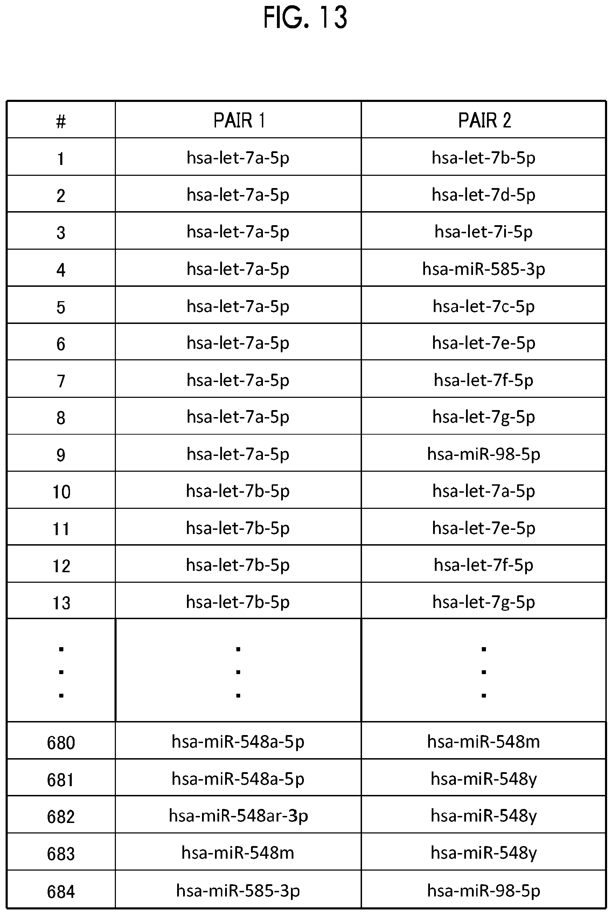

Regarding the interaction between the measurers, the interaction between 684 pairs of the measurers is specified as a result of the studies by the method of the evaluation step (step S12). Fig. 13 is a table showing combinations of the measurers whose interaction is specified. Two miRNAs whose interaction is specified are described in each of Pair 1 and Pair 2.

The selection of the feature amount set that can be measured at the same time is attempted by the method of the embodiment. As a result, it is possible to specify 79 types of miRNAs, which is 45% of the 177 types, that can be measured at the same time as the measurable feature amount set. That is, it can be understood that the method capable of measuring as many miRNAs as possible at the same time with only one tube (one measurement) can be proposed.

### Explanation of References

10: apparatus
20: feature amount candidate
22: simultaneous unsuitable marked graph
24: feature amount independent set
26: measurable suitable feature amount
32: feature amount data set
34: feature amount candidate
36: measurable feature amount candidate
38: measurable suitable feature amount
40: feature amount selection candidate
42: measurable suitable feature amount
100: processing unit
110: design unit
112: evaluation unit
114: data structure construction unit
116: extraction unit
118: selection unit
120: operation unit
122: confirmation unit
123: output unit
125: display control unit
130: CPU
135: ROM
140: RAM
200: storage unit
300: display unit
310: monitor
400: input unit
410: keyboard
420: mouse
500: external server
510: external database
602: feature amount data set
604: feature amount candidate
606: measurable feature amount

## Claims

1. A method of selecting one or a plurality of feature amounts that are used to predict or discriminate a characteristic of a sample and designing one or a plurality of measurers that measure the feature amount, the method performed by a processor comprising:
a step of attempting to design the measurer for a feature amount candidate;
an evaluation step of evaluating interaction between the designed plurality of measurers;
a data structure construction step of associating, as a combination pair of the measurers that are unable to be set at the same time, a combination of the measurers whose interaction evaluated in the evaluation step is problematic to construct a graph-like data structure;
an extraction step of selecting a portion corresponding to one or a plurality of independent sets from the graph-like data structure to extract one or more feature amount set candidates that do not include the combination pair whose interaction is problematic; and
a selection step of selecting, as a feature amount set to be measured, the feature amount set candidate or a subset of the feature amount set candidate.

2. The method according to claim 1,
wherein, in the extraction step, one or a plurality of maximal independent sets are selected among the plurality of independent sets to extract one or more feature amount set candidates,
next to the selection step, the method further comprising:
an operation step of performing an operation including feature amount selection for each of the selected feature amount set candidates; and
a confirmation step of evaluating a result of the operation step to confirm a feature amount set to be measured based on a result of the evaluation.

3. The method according to claim 1 or 2,
wherein, in the step of attempting to design the measurer, the design of the plurality of measurers is attempted for at least one or more of the feature amount candidates, respectively, and
in the data structure construction step, all the plurality of measurers are considered and any measurer pair for the same feature amount candidate is associated as the combination pair that is unable to be set at the same time.

4. The method according to any one of claims 1 to 3,
wherein the graph-like data structure includes a graph data structure or a data structure conforming to a graph.

5. The method according to any one of claims 1 to 4,
wherein the feature amount is a gene, and the measurer is a primer.

6. The method according to claim 5,
wherein the interaction between the measurers is a primer dimer.

7. The method according to claim 5 or 6,
wherein the gene is a methylation DNA site or a miRNA.

8. An apparatus comprising:
a processor that selects one or a plurality of feature amounts that are used to predict or discriminate a characteristic of a sample and designs one or a plurality of measurers that measure the feature amount,
wherein the processor is configured to:
attempt to design the measurer for a feature amount candidate;
evaluate interaction between the designed plurality of measurers;
associate, as a combination pair of the measurers that are unable to be set at the same time, a combination of the measurers whose evaluated interaction is problematic to construct a graph-like data structure;
select a portion corresponding to one or a plurality of independent sets from the graph-like data structure to extract one or more feature amount set candidates that do not include the combination pair whose interaction is problematic; and
select, as a feature amount set to be measured, the feature amount set candidate or a subset of the feature amount set candidate.

9. The apparatus according to claim 8,
wherein the processor is configured to:
select one or a plurality of maximal independent sets among the plurality of independent sets to extract one or more feature amount set candidates;
perform an operation including feature amount selection for each of the extracted feature amount set candidates; and
evaluate a result of an operation step to confirm a feature amount set to be measured based on a result of the evaluation.

10. The apparatus according to claim 8 or 9,
wherein, in a case of attempting to design the measurer, the design of the plurality of measurers is attempted for at least one or more of the feature amount candidates, respectively, and
all the plurality of measurers are considered and any measurer pair for the same feature amount candidate is associated as the combination pair that is unable to be set at the same time to construct the graph-like data structure.

11. A program that selects one or a plurality of feature amounts that are used to predict or discriminate a characteristic of a sample and designs one or a plurality of measurers that measure the feature amount, the program causing a computer to execute:
a step of attempting to design the measurer for a feature amount candidate;
an evaluation step of evaluating interaction between the designed plurality of measurers;
a data structure construction step of associating, as a combination pair of the measurers that are unable to be set at the same time, a combination of the measurers whose interaction evaluated in the evaluation step is problematic to construct a graph-like data structure;
an extraction step of selecting a portion corresponding to one or a plurality of independent sets from the graph-like data structure to extract one or more feature amount set candidates that do not include the combination pair whose interaction is problematic; and
a selection step of selecting, as a feature amount set to be measured, the feature amount set candidate or a subset of the feature amount set candidate.

12. A non-transitory computer-readable recording medium on which the program according to claim 11 is recorded.
